(19) **Europäisches Patentamt / European Patent Office / Office européen des brevets**

(11) **EP 1 932 497 A1**

(12) **EUROPEAN PATENT APPLICATION**
published in accordance with Art. 153(4) EPC

(43) Date of publication:
**18.06.2008 Bulletin 2008/25**

(21) Application number: **06811231.7**

(22) Date of filing: **04.10.2006**

(51) Int Cl.:
**A61F 2/84** (2006.01)

(86) International application number:
**PCT/JP2006/319892**

(87) International publication number:
**WO 2007/040250 (12.04.2007 Gazette 2007/15)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.10.2005 JP 2005292799**

(71) Applicant: **Kaneka Corporation Kita-ku Osaka-shi, Osaka 530-8288 (JP)**

(72) Inventor: **NAKANO, Ryoji Settsu-shi, Osaka 566-0072 (JP)**

(74) Representative: **Gille Hrabal Struck Neidlein Prop Roos Patentanwälte Brucknerstrasse 20 40593 Düsseldorf (DE)**

(54) **STENT TO BE PLACED IN THE LIVING BODY**

(57) It is intended to provide a stent to be placed in the living body which shows a low restenosis rate after being placed in the body. A stent to be placed in the living body **characterized in that** the stent is in a long and thin almost tubular shape having both termini and provided with voids, the long and thin almost tubular body can be extended in the radial direction from the first diameter in the compressed state to the second diameter in the extended state, the strut section of the stent is in an almost square or rectangular shape, the whole surface of the stent has an outer surface, an inner surface and a side surface, and the side surface area corresponding to the area of the side surface is not more than 40% of the whole surface area corresponding to the area of the whole surface of the stent.

Fig. 2

21  24  23  25  23

EP 1 932 497 A1

**Description**

TECHNICAL FIELD

The present invention relates to a method of preventing and treating exuberant vascular proliferation and a medical stent for placement in body used for that purpose.

Cross-Reference to Related Applications

The disclosure in the description, claims, drawings, and abstract of Japanese Patent Application No. 2005-292799 (filed on Oct. 5, 2005) is hereby incorporated by reference in its entirety.

BACKGROUND ART

The stent is a medical device that is placed in blood vessel or other lumen in the body and that is used for dilation of its stricture or obstruction site and preservation of the lumen size and thus for treatment of various diseases caused by constriction or obstruction of blood vessel or other internal lumens. Examples of such stents include stents in the coiled shape of a single linear metal or polymeric material, those prepared by processing a metal tube with laser, assemblies of linear parts welded to each other with laser, those prepared by weaving multiple linear metal wires, and the like.

These stents are grouped into those expanded by balloon (balloon-expandable stents) and those expandable as they are when an external part restricting expansion is removed (self-expandable stents). Such a balloon-expandable stent is expanded and fixed to the lumen to be treated, as it is fixed to the balloon region of an intravascular catheter having an expandable part such as balloon at the distal end (balloon catheter) (in mounting step), the catheter fed to the site in the patient lumen to be treated, and the balloon expanded in the treatment site. Subsequently, the balloon is contracted, and the catheter withdrawn. In expanding the balloon, the expansion pressure is adjusted according to the condition of the lumen to be expanded and the mechanical strength of the stent used.

The stent is used, for example, as a method of preventing or treating exuberant vascular proliferation, and thus, the purpose thereof is to prevent obstruction of internal blood vessel by proliferation. Demanded for such a stent are various properties such as strength sufficient for overcoming the pressure by the tubular organ to be expanded, flexibility allowing supply of the stent through a highly winding tubular organ to a desired site without problem, post-expansion flexibility preventing damage on the tubular organ during and after placement in tubular organ, evenness of expansion and fineness of design allowing uniform coverage of the tubular organ, and non-X ray permeability allowing the surgeon to identify the desired location during catheter placement operation by X ray monitoring. For the purpose of satisfying these requirements, various stent designs were proposed, as disclosed, for example, in Patent Documents 1 and 2.

Recently, these stents are used more frequently in angioplasty of heart and carotid artery, and, although it was shown that placement of such a stent was effective in reducing the frequency of restenosis statistically significantly, the frequency of restenosis still remains high even now. For example in the case of cardiac coronary artery, it was reported that stent placement resulted in restenosis at a frequency of approximately 20 to 30%. Restenosis is induced both by biological vascular damage and also by vascular damage due to stent placement. Typical vascular constriction-restenosis induced by vascular damage is considered to be caused by proliferation of the intimal smooth muscle cells. First, the vascular damage induces proliferation of the smooth muscle cells, and the proliferated smooth muscle cells migrate into the inner membrane. Subsequently, the smooth muscle cells in the inner membrane proliferate with substrate deposition, thickening the inner membrane.

For example, Patent Document 3 proposes application of an obstruction-preventing drug on stent for reduction of restenosis rate. Examples of the obstruction-preventing drugs discussed include anticoagulant agents, antiplatelet agents, anticonvulsant agents, antibacterial agents, anti-tumor drugs, antimicrobial agents, antiinflammatory agents, anti-metabolism agents, immunosuppressive agents, and the like. Also proposed was a method of reducing restenosis by coating on stent an immunosuppressive agent, such as cyclosporine, tacrolimus (FK-506), Sirolimus (rapamycin), mycophenolate mofetil, or the analogue thereof. Specifically for example, Patent Document 4 discloses a stent coated with an immunosuppressive agent Sirolimus (rapamycin), while, for example, Patent Document 5 discloses a stent coated with an anti-tumor drug taxol (paclitaxel). For example, Patent Documents 6 and 7 disclose stents coated with tacrolimus (FK-506). However, there is currently, still restenosis after stent placement occurring at a certain rate even when such a medicine-coated stent is used, and there is a need for optimization of the basic stent design for further reduction of the restenosis rate.

Patent Document 1: Japanese Unexamined Patent Publication No. 2-174859
Patent Document 2: Japanese Unexamined Patent Publication No. 6-181993

Patent Document 3: Japanese Unexamined Patent Publication No. 5-502179
Patent Document 4: Japanese Unexamined Patent Publication No. 6-9390
Patent Document 5: Japanese Unexamined Patent Publication No. 9-503488
Patent Document 6: WO 02/065947
Patent Document 7: EP Patent No. 1254674

Disclosure of the Invention

Problems to be Solved by the Invention

An object of the present invention, which was made under the circumstance above, is to provide a stent for placement in body giving a lower restenosis rate after stent placement.

Means to Solve the Problems

The present invention has the following one and more aspects.

(1) An aspect of the present invention is a stent for placement in body, characterized in that: the stent is a long tube-shaped net having openings and end regions; the long tube-shaped net is expandable in the radial direction from a compressed first diameter to a second expanded diameter; the strut cross-section of the stent is almost square or rectangle; and, when the entire stent surface includes external surface, internal surface, and side surface, the surface area of the side surface is 40% or less of the entire surface area of the stent.
(2) In a favorable embodiment of the invention, the long tube-shaped net has multiple almost wave-formed cylindrical elements aligned in the axial direction.
(3) In another favorable embodiment, the material for the stent is a metal containing cobalt.
(4) In yet another favorable embodiment, a drug preventing vascular obstruction is fixed to the external surface of the stent.
(5) In yet another favorable embodiment, the material for the stent is a biodegradable polymer.

The aspects (1) to (5) may be worked in combination of part or all of them. The characteristics and the advantageous effects of the present invention including those described above will be more obvious, when the invention is described with reference to the following embodiments and drawings.

Advantageous effects of the invention

The present invention provides a stent for placement in body giving a low restenosis rate after stent placement.

BRIEF DESCRIPTION OF THE DRAWINGS

Figure 1 is a schematic view showing a commonly-known stent for placement in body.
Figure 2 is a development view showing the stent for placement in body in an embodiment of the present invention.

EXPLANATION OF REFERENCES

11   Stent external surface
12   Stent internal surface
13   Stent side surface
21   first end section
22   Second end section
23   Third central section
24   Fourth section
25   Fifth section

BEST MODE OF CARRYING OUT THE INVENTION

Hereinafter, favorable examples of the stent according to the present invention will be described, but the invention is not restricted by these examples.

1. Basic shape

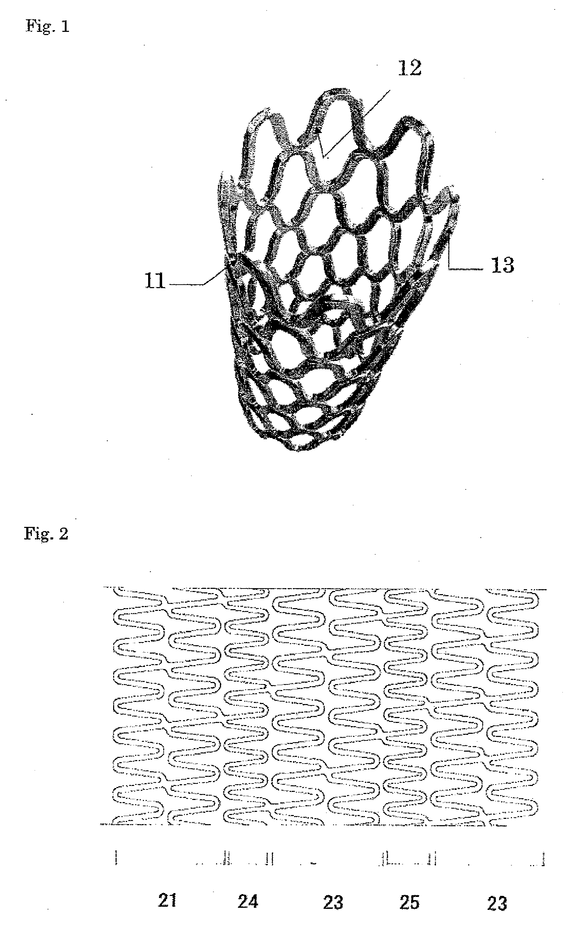

A development view of the stent for placement in body in an embodiment of the present invention is shown in Figure 2. The stent exemplified in Figure 2 has a stent for placement in body, characterized in that, the stent is a long tube-shaped net having openings and end regions, the long tube-shaped net is expandable in the radial direction from a compressed first diameter to a second expanded diameter, the strut cross-section of the stent is almost square or rectangular, and, when the entire stent surface includes external surface, internal surface, and side surface, the surface area of the side surface is 40% or less of the entire surface area of the stent.

For example, the stent shown in Figure 2 has a first end section 21, a second end section 22 and a third central section 23 as the cylindrical shape elements, each of which has eight wave-formed bents peripherally, and a fourth section 24 and a fifth section 25 also as the cylindrical shape elements, each of which has 10 wave-formed bents peripherally. Each cylindrical shape element has multiple wave-formed bents aligned in the axial direction of the stent (long tube-shaped net).

The term "wave-formed" means that the shape is close to the shape of common sine wave, and also includes waveforms such as square, triangular, and saw-shaped forms. All wave-formed bents included in the wave-formed elements may have an identical waveform or multiple waveforms different in amplitude, width, or shape.

2. Stent surface

Considering the influence on blood flow after placement of a stent, the surface area of the side surface is preferably smaller. For preservation of the blood flow in the blood vessel by resisting the external force from the blood vessel, the side surface area is preferably larger. From these viewpoints, the surface area of the side surface should be 40% or less of the entire surface area of the stent. In addition, the side surface area is preferably 30% or more, from the viewpoint of productivity

The entire surface area here is that of the stent, specifically the sum of the external surface area, the internal surface area, and the side surface area. The external surface is the surface of the peripheral face of the almost cylindrical stent, i.e., the face directly in contact with the body when the stent is placed and expanded in the body. The internal surface is the surface of the internal face of the almost cylindrical stent, i.e., the face in contact with the catheter when it is crimped to the stent catheter. The side surface is the surface of the almost cylindrical stent except the external and internal surfaces, i.e., the face normal to the stent axial direction. In the general embodiment of the stent for placement in body shown in Figure 1, 11 represents the external surface, 12 the internal surface, and 13 the side surface.

The side surface area and the entire surface area are determined in the following manner. The strut constituting stent is normally surface-smoothened and rounded at corners for reduction of damage on body organs. The surface area in the present invention is a value determined from the developed stent image obtained as will be described below without any consideration to the rounding, and the area of the region facing outward is the external surface area; the product of the all peripheral length of the region facing outward multiplied by the thickness, the side surface area; and a value obtained by dividing the external surface area by the stent external diameter and multiplying the value by the stent internal diameter, the internal surface area.

The stent image is obtained in the following manner. An image of external surface of an almost cylindrical stent is prepared, and the external surface area and the length of the stent peripheral region of the external surface are determined. Separately, the thickness of the stent is determined by using a thickness gage, and the stent entire area and the stent side surface area are calculated from the external surface area, the stent region peripheral length and the thickness by simple calculation. The image of the external surface of the almost cylindrical stent can be obtained easily in combination of a line-scan camera, a high-accuracy stent-rotating device, and a light source installed in the almost cylindrical stent.

3. Stent-producing method

The stent-producing method for use may be any one of stent-producing methods commonly practiced such as laser processing, electric-discharge machining, mechanical machining, and etching. Surface smoothening of the strut terminal regions, by various polishing methods such as electropolishing, after stent production is well known in the art, and such a method is also applicable in the present invention.

The first diameter of the compressed stent is set to, for example, 1.2 mm or less, preferably 0.9 mm or less.

The second diameter of the expanded stent, which is determined according to the internal diameter of the patient lumen, varies, depending on the lumen to be treatment. For example in the case of cardiac coronary artery, the second diameter is set to approximately 2.0 mm to 5.0 mm.

The stent length depends on the length of the area of the patient lumen to be treated. For example in the case of vascular system, a stent having a diameter of approximately 7 mm to 100 mm is used, while in the case of cardiac coronary artery, a stent having a diameter of approximately 7 to 40 mm is used.

### 4. Structural material

Metal materials favorable for the structural material include stainless steel, titanium, nickel, iridium, oxidation iridium magnesium, niobium, platinum, tantalum, gold, and the alloys thereof, as well as gold-plated iron alloys, platinum-plated iron alloys, cobalt chromium alloys, and titanium nitride-coated stainless steel. Favorably from the viewpoints of favorable rigidity and elasticity, the stent according to the present invention is made of a metal such as stainless steel, a nickel alloy such as Ni-Ti alloy, a Cu-Al-Mn alloy, or a Co-Cr alloy, or a combination thereof, and, for example, the metals specified in JIS-G4303 or the metals specified in ISO5832-5, ISO5832-6, and ISO5832-7 can also be used. For prevention of X ray permeation, the material is preferably a metal containing cobalt.

### 5. Biocompatible polymer

Any biocompatible polymer may be used as the biocompatible polymer, if it is resistant to deposition of platelets and not irritative to organs and allows release of drugs. Examples of favorable synthetic polymers include polyether-type polyurethanes, blends or block copolymers of dimethylsilicone, polyurethanes such as segmented polyurethanes, polyacrylamides, polyethyleneoxides, polycarbonates such as polyethylene carbonate and polypropylene carbonate, and the like. Examples of natural biocompatible polymers include fibrin, gelatin, collagen, and the like. These polymers may be used alone or in combination of two or more as needed.

### 6. Biodegradable polymer

Any biodegradable polymer may be used as the biodegradable polymer, if it is decomposed enzymatically or non-enzymatically in the body, gives non-toxic decomposition products, and can release drugs. An example thereof is a compound properly selected from polylactic acid, polyglycol acid, copolymers of polylactic acid and polyglycolic acid, collagen, gelatin, chitin, chitosan, hyaluronic acid, polyamino acids such as poly-L-glutamic acid and poly-L-lysine, starch, poly-$\varepsilon$-caprolactone, polyethylene succinate, poly-$\beta$-hydroxy alkanoate, and the like. These polymers may be used alone or in combination of two or more.

### 7. Drug

Drugs preventing vascular obstruction include various drugs such as anticoagulant agents, antiplatelet agents, anticonvulsant agents, antibacterial agents, anti-tumor drugs, antimicrobial agents, antiinflammatory agents, anti-metabolism agents, immunosuppressive agents, and the like. Examples of the immunosuppressive agents include cyclosporine, tacrolimus (FK-506), Sirolimus (rapamycin), mycophenolate mofetil and the analogues thereof.

Such a drug suppressing vascular obstruction is fixed to the stent external surface, for example, by dipping or coating.

EXAMPLES

Hereinafter, the stent according to the present invention will be described in detail with reference to Examples, but it should be understood that the present invention is not restricted thereby.

An example of the method of placing a stent is to fix the stent as it is compressed into the balloon region at the distal end of a catheter, feed the catheter and the stent into the patient lumen to be treated, fix the stent by expanding the balloon, and then, withdraw the catheter. Thus, the stent has two states: compressed state and expanded state. The stent is delivered in the compressed state and placed in a patient lumen in the expanded state. The stents in Comparative Examples and Examples shown below were prepared by a production method known in the art, i.e., by cutting a raw cylindrical metal tube into the shape of stent by laser cutting and additionally polishing the surface electrolytically

Figure 1 is a schematic view of a commonly-known stent for placement in body, as seen from an inclined direction. The stent for placement in body shown in Figure 1 is a long tube-shaped net having two end regions and cells of multiple wave-formed bents that are aligned to form an almost cylindrical tube between the end regions.

Figure 2 is a development view showing a structure of the long tube-shaped net of a stent for placement in body having two end regions in an embodiment of the present invention. The stent for placement in body shown in Figure 2 has multiple cylindrical shape elements; each of the cylindrical shape elements consists of almost wave-formed elements; these cylindrical shape elements are expandable in the radial direction and connected to each other substantially in a well-aligned manner about the common longitudinal direction axis line by connecting some of the wave-top regions of the almost wave-formed elements each other.

As shown in Figure 2, in the first end section 21, the second end section 22 and the third central section 23, the cylindrical shape element has eight wave-formed bents peripherally, while in the fourth section 24 and the fifth section 25, it has 10 wave-formed bents peripherally. These cylindrical shape elements are bound to each other about the longitudinal direction axis line. Separately, a stent having a stent length of 10.30 mm and a stent external diameter of 1.80 mm immediately after production was prepared by using the cobalt chromium alloy specified in IS05832-7. The fourth section 24 and fifth section 25 are located at positions respectively 2.70 mm to 3.75 mm separated from the stent ends.

The strut width of the wave shape elements in the first end section 21, the second end section 22, and the third terminal section 23 are respectively selected in the range of 120 to 130 $\mu$m, and the strut width thereof in the fourth section 24 and the fifth section 25 in the range of 100 to 110 $\mu$m, and the stent thickness in the range of 60 to 120 $\mu$m; and thus, six kinds of stents for placement in body were prepared in Comparative Examples and Examples. The total length and the surface area of the six kinds of stents thus prepared are summarized in the following Table 1. The development views of the stents in Comparative Examples and Examples were similar to that shown in Figure 2.

Similarly, by using the stent design shown in Figure 2 a stent having a stent thickness of 50 $\mu$m was prepared without success, because the thickness was too low.

As described above, multiple stents for placement in body, different in the width and the thickness of the strut constituting the stent but identical in the stent design, were prepared.

Then, the entire surface area and the side surface area of the stents for placement in body thus prepared were determined, and the rate of the side surface area to the entire surface area calculated.

The rates of the side wall surface area to the entire area of the stents prepared by Comparative Examples 1 to 3 and Examples 1 to 3 are summarized in the following Table 1.

[Table 1]

|  | Stent total length | Stent shape | Rate of side wall surface area to entire surface area |
|---|---|---|---|
| Comparative Example 1 | 10.30mm | Figure2 | 51.4% |
| Comparative Example 2 | 10.30mm | Figure2 | 47.0% |
| Comparative Example 3 | 10.30mm | Figure2 | 43.8% |
| Example 1 | 10.30mm | Figure2 | 39.1% |
| Example 2 | 10.30mm | Figure2 | 36.3% |
| Example 3 | 10.30mm | Figure2 | 33.8% |

(Evaluation of vascular obstruction rate)

The stents prepared in Comparative Examples 1 to 3 and Examples 1 to 3 were evaluated in the following manner. First, each of the stents for evaluation is compressed and fixed into the balloon region of a balloon catheter. The balloon catheter used then was a rapid exchange balloon catheter having a balloon diameter of 3.0 mm and a balloon region length of 13 mm at the rated expansion pressure.

Subsequently, stent placement experiments were carried out by using small pigs (Crown, female, 8 to 12 months of age) for evaluation. A sheath (6Fr) was inserted into the right femoral artery of a small pig under anesthesia, and the distal end of a guiding catheter (6Fr) inserted through the sheath was engaged into the entrance to the left coronary artery. A stent was delivered along the guiding catheter to the left coronary artery frontal descending branch and the left coronary artery circumflex branch and placed there as it is expanded. After withdrawal of the guiding catheter and the sheath, the right femoral artery was bound tightly for homeostasis. The stent was placed in the stent placement region, while the stent diameter/blood vessel diameter ratio was adjusted to approximately 1.25. A stent was left in each blood vessel of left coronary artery frontal descending branch, left coronary artery circumflex branch, and right coronary blood vessel. There was no problem of difficulty in placing the stent because of excessively smaller vascular diameter.

A mixture of aspirin (330 mg/day) and ticlopidine (250 mg/day) was administered to the pig from a day before stent placement to the day of anatomical examination. The small pig was put to sleep 28 days after placement, and the heart was removed. A stent was placed in a small pig in each Comparative Example 1 to 3 or Example 1 to 3 (n=1) and thus, a total of six stents were placed. There was no problem in placing the stent in all tests, and there was also no problem such as stent obstruction during the placement period for 28 days.

The coronary artery of stent placement was removed from the heart and immersed and fixed in 10% neutral buffered formalin solution. After resin embedding, each stent was cut at three points, specifically at the center and the points 1.5 mm from both ends, giving a total of three sections, which were subjected to H.E. staining (haematoxylin-eosin staining) and E.V.G. staining (Elastica-van Gieson staining), and the degree of vascular obstruction of the stents was compared. The vascular lumen area (LA: Lumen Area) and the area within the internal elastic lamina (IELA) of the sections in each stent were determined. The vascular obstruction rate was calculated from the vascular lumen area (LA) and the area within the internal elastic lamina (IELA) according to the following Formula:

$$\text{Vascular obstruction rate (\%)} = (1 - (LA/IELA)) \times 100$$

The data at the center and the points 1.5 mm from both ends were obtained, and the average thereof was used as the data for the stent.

[Table 2]

|  | Average vascular obstruction rate |
| --- | --- |
| Comparative Example 1 | 43.6% |
| Comparative Example 2 | 41.2% |
| Comparative Example 3 | 41.1% |
| Example 1 | 32.8% |
| Example 2 | 34.6% |
| Example 3 | 33.5% |

As shown in Table 2, the vascular obstruction rates in Examples 1 to 3 were found to be lower than those in Comparative Examples 1 to 3. The vascular obstruction rate obtained in Example 1, the lowest rate of all Examples, was lower by 20.2% than that obtained in Comparative Example 3, the lowest rate in all Comparative Examples, which was a significantly large reduction in vascular obstruction rate. Among Examples 1 to 3, the vascular obstruction rate in Example 1 was the lowest, but not significantly different from those in other Examples.

**Claims**

1. A stent for placement in body, **characterized in that**:

   the stent is a long tube-shaped net having openings and end regions;
   the long tube-shaped net is expandable in the radial direction from a compressed first diameter to a second expanded diameter;
   the strut cross-section of the stent is almost square or rectangular; and when the entire stent surface includes external surface, internal surface, and side surface, the surface area of the side surface is 40% or less of the entire surface area of the stent.

2. The stent for placement in body according to Claim 1, wherein the long tube-shaped net has multiple almost wave-formed cylindrical elements aligned in the axial direction.

3. The stent for placement in body according to Claim 1 or 2, wherein the material for the stent is a metal containing cobalt.

4. The stent for placement in body according to any of Claims 1 to 3, wherein a drug preventing vascular obstruction is fixed to the external surface of the stent.

5. The stent for placement in body according to any of Claims 1 to 4, wherein the material for the stent is a biodegradable polymer.

Fig. 1

Fig. 2

## INTERNATIONAL SEARCH REPORT

| | International application No. |
|---|---|
| | PCT/JP2006/319892 |

**A. CLASSIFICATION OF SUBJECT MATTER**
*A61F2/84(2006.01)i*

According to International Patent Classification (IPC) or to both national classification and IPC

**B. FIELDS SEARCHED**

Minimum documentation searched (classification system followed by classification symbols)
A61F2/84

Documentation searched other than minimum documentation to the extent that such documents are included in the fields searched
Jitsuyo Shinan Koho 1922-1996 Jitsuyo Shinan Toroku Koho 1996-2006
Kokai Jitsuyo Shinan Koho 1971-2006 Toroku Jitsuyo Shinan Koho 1994-2006

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-527279 A (Conor Medsystems, Inc.),<br>09 September, 2004 (09.09.04),<br>Par. No. [0009]<br>& US 6964680 B2 & EP 1359867 A2 | 1<br>2-5 |
| Y | JP 11-221288 A (Cordis Corp.),<br>17 August, 1999 (17.08.99),<br>Par. Nos. [0019] to [0022], [0034] to [0035]<br>& US 5913897 A & EP 0910998 A2 | 2,4 |
| Y | JP 2004-344469 A (Terumo Corp.),<br>09 December, 2004 (09.12.04),<br>Par. Nos. [0014], [0023]<br>(Family: none) | 3,5 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 November, 2006 (02.11.06) | 14 November, 2006 (14.11.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

# EP 1 932 497 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005292799 A
- JP 2174859 A
- JP 6181993 A
- JP 5502179 A
- JP 6009390 A
- JP 9503488 A
- WO 02065947 A
- EP 1254674 A